(19) Europäisches Patentamt
European Patent Office
Office européen des brevets

(11) Publication number: 0 197 757
A2

(12) # EUROPEAN PATENT APPLICATION

(21) Application number: 86302438.6

(22) Date of filing: 02.04.86

(51) Int. Cl.[4]: C 07 D 519/00
A 61 K 31/35
//(C07D519/00, 493:00, 493:00)

(30) Priority: 09.04.85 US 721360

(43) Date of publication of application:
15.10.86 Bulletin 86/42

(84) Designated Contracting States:
AT BE CH DE FR GB IT LI LU NL SE

(71) Applicant: PFIZER INC.
235 East 42nd Street
New York, N.Y. 10017(US)

(72) Inventor: Moore, Bernard Shields
39, Savi Avenue
Waterford Connecticut(US)

(74) Representative: Wood, David John et al,
Pfizer Limited Ramsgate Road
Sandwich Kent CT13 9NJ(GB)

(54) **Crystalline anhydrous sodium 19-deoxyaglycone dianemycin.**

(57) A process for preparing a crystalline, anhydrous sodium salt of –19–deoxyaglycone dianemycin by precipitation from a dry methylene chloride-hexane solution.

EP 0 197 757 A2

# CRYSTALLINE ANHYDROUS SODIUM 19-DEOXYAGLYCONE DIANEMYCIN

19-Deoxyaglycone dianemycin is an ionophore antibiotic described by Celmer, et al. in U.S. Patent No. 4,431,801 and Westley, et al., J. Antibiotics, 37, 813 (1984). This compound, which is active against coccidiosis, enteritis, swine dysentery and theileriosis, as well as being effective in promoting growth in poultry and ruminants, has the following chemical structure:

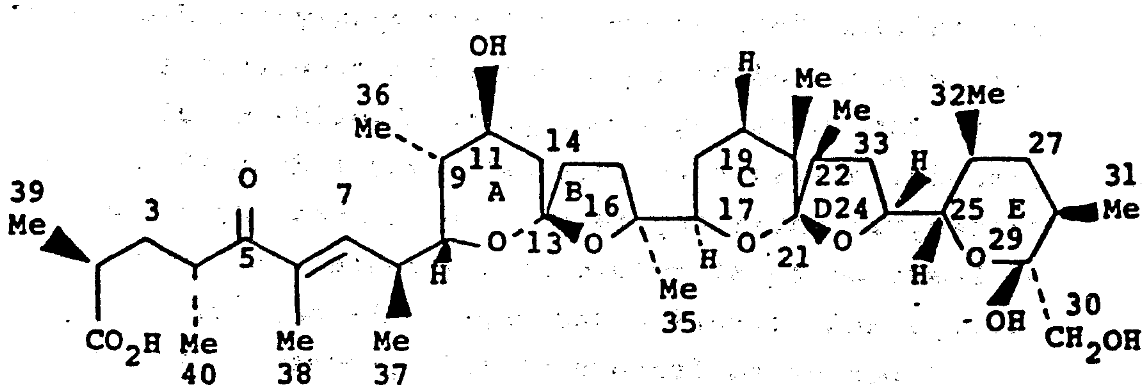

19-Deoxyaglycone dianemycin is isolated from Streptomyces halstedii ATCC 31812 as the sodium salt at a level of purity of aboout 90% based on biological activity. The salt can be purified by recrystallization from a number of different solvents. If recrystallized without regard for solvent purity it crystallizes as a hydrate. While the recrystallized product appears to be relatively pure by high performance liquid chromatography, it's crystals, by X-ray and differential scanning calorimetry, are not uniform and it's melting point is variable. Vacuum drying of the crystalline hydrate gives an amorphorus solid. In addition, the use of certain organic solvents in recrystallization provides a hydrate which is amorphous and hygroscopic.

Thus, the physical forms of sodium 19-deoxyaglycone dianemycin known heretofore exhibit characteristics which are unacceptable in a drug, as they make pharmaceutical compounding of said drug a difficult task. Further, the absence of consistant characterizing features, such as melting point, crystal structure, differential scanning calorimeter, etc. makes the routine task of assaying sodium 19-deoxyaglycone dianemycin for purity extremely difficult, if not impossible.

It has now been found that a stable anhydrous, crystalline form of sodium 19-deoxyaglycone dianemycin can be prepared by a process which comprises the steps of a) concentrating a methylene chloride solution containing less than .05% water and sodium 19-deoxyaglycone dianemycin until crystallization commences; b) adding at least an equal volume of dry hexane to the resulting slurry; and filtering and drying the solids.

A preferred feature of this process is the concentration of the methylene chloride by heating above the boiling point at atmospheric pressure.

Also included as part of the present invention is crystalline, anhydrous sodium 19-deoxyaglycone dianemycin.

The process of the present invention is comprised of several steps, the first of which is the concentrating of a dry methylene chloride solution of sodium 19-deoxyaglycone dianemycin to a point where crystallization of said sodium salt commences.

Since sodium 19-deoxyaglycone dianemycin has a tenacity for water in an organic solvent, usually crystallizing as the monohydrate, it is necessary the methylene chloride -sodium 19-deoxyaglycone dianemycin solution be dry. Accordingly, a methylene chloride -sodium 19-deoxyaglycone dianemycin solution containing

less than .05% water should be employed if an anhydrous form of this sodium salt is to be obtained. Removal of the water from the methylene chloride -sodium 19-deoxyaglycone dianemycin solution can be achieved by several means, such as drying with chemical drying agents, as sodium sulfate, magnesium sulfate, etc; other methods of drying which employ molecular sieves or azeotroping of residual water can also be employed.

It is preferred that the methylene chloride be concentrated by heating at atmospheric pressure. Sufficient heat should be applied to cause the solvent to boil. Alternate methods for concentration can be employed, such as evaporating at room temperature under a stream of inert gas in a moisture free system or concentrating the solution under vacuum with heating below the boiling point of the solvent.

When the sodium 19-deoxyaglycone dianemycin starts to crystallize from the methylene chloride the second step of this process is carried out and hexane is added to the slurry or mixture to ensure as complete recovery as possible of the desired product from the methylene chloride. It has also been found that hexane not only aids in the crystallization of the product from methylene chloride, but does not interfere with the type of crystal formation initially started on concentration of the methylene chloride solution. It is necessary that the hexane employed also be dry, and contain less than .05% water, as can be achieved by the use of any one of the aforementioned drying techniques.

The amount of hexane employed is not critical, although sufficient amounts should be employed to ensure that as much of the sodium 19-deoxyaglycone dianemycin as possible is crystallized from the solvent mixture. At least a volume of hexane equal to that of the methylene chloride, after crystallization has

commenced, is sufficient to facilitate recovery of most of the desired product. Lesser amounts result in poorer recovery. Preferably, a 2-4 fold excess of hexane is used.

Following crystallization, the product is filtered and dried. When filtering it is desirable, in order to avoid excessive amounts of water condensing on the filtered product, to minimize the amount of air pulled over the filtered solids. Filtration under a blanket of dry nitrogen can also protect the cake from excess moisture. The filtered, hexane-damp solids can be dried in a drying oven at atmospheric or, more efficiently, at elevated temperatures under vacuum.

The crystals obtained are brittle rods, relatively large in form, thus lending themselves to easy milling. Karl Fisher water measurements on the filtered solids indicate they contain about 0.2 to 0.7% water. The melting point of the crystals are very sharp and they exhibit a single sharp endotherm by differential scanning calorimetry.

The following examples are provided solely for the purpose of further illustration.

Example 1

To a solution of 1960 g of the sodium salt of 19-deoxyaglycone dianemycin (88.2% activity) in 23.5 liters of methylene chloride was added 1.2 kg of 4A molecular sieves and the resulting mixture allowed to stir at room temperature for one hour. The mixture was filtered through #2 Whatman paper and 784 g of celite, and the celite-sieve cake washed with 8 liters of methylene chloride. The combined filtrate and washings were concentrated atmospherically at steam bath temperatures to about 3.8 liters at which time a crystalline precipitate started to form. An additional 500 ml of solvent was removed, the heat was withdrawn and 11 liters of hexane were added in a steady stream with stirring. The mixture was allowed to stir for one hour and the crystalline product filtered, washed with 8 liters of dry hexane and dried at 50°C and 1 mm Hg, 1566 g (90.6% yield), mp 215.5-217.5°C, water content 0.24% (KF) and differential scanning calorimeter (DCS) 225.88°C (endotherm 9.75 cal/g).

Example 2

Ten grams of the sodium salt of 19-deoxyaglycone dianemycin (88.2% activity) was dissolved in 100 ml of methylene chloride followed by the addition of 7 g of 4A molecular sieves. After stirring for one hour the mixture was filtered through 4 g of celite and the residue washed with 50 ml of methylene chloride. The washings and filtrate were combined and concentrated on a steam bath to about 20-25 ml at which point the product was crystallizing. The heat was removed and 60 ml of dry hexane was added to the slurry. The mixture was stirred for 2.4 hours and the product was filtered, washed with 50 ml of dry hexane and vacuum dried at 50°C and 3 mm pressure, 7.8 g (88.3% yield), mp 216.5-218°C, water content 0.68% (KF) and DSC 225.04°C.

0197757

CLAIMS for the contracting States BE, CH, DE, FR, GB, IT, LI, LU, NL, SE.

1. The crystalline, anhydrous sodium salt of the compound of the formula

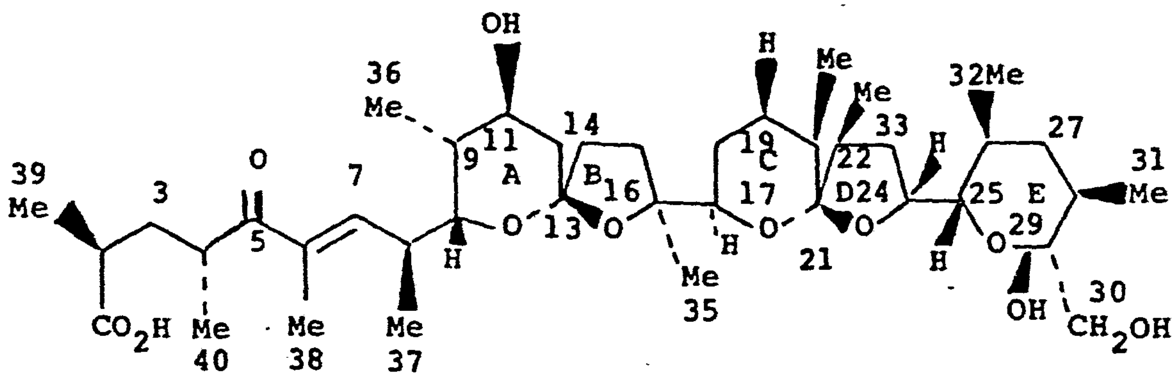

2. A process for preparing the compound of claim 1, comprising the steps of a) concentrating a methylene chloride solution containing <.05% water and the sodium salt of a compound of the formula

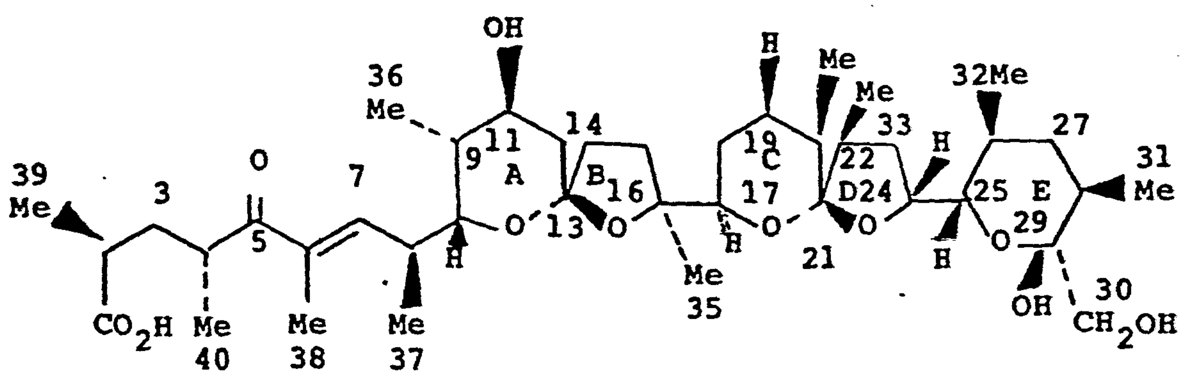

until crystallization commences; b) adding at least an equal volume of dry hexane containing <.05% water to the resulting slurry; and c) filtering and drying the solids.

3. The process of claim 2, wherein the methylene chloride solution is concentrated by heating above the boiling point at atmospheric pressure.

4. A crystalline anhydrous sodium salt as claimed in claim 1 for use as a medicament,

0197757
P.C. 6936

CLAIMS for the Contracting State AT

1. A process for preparing the crystalline, anhydrous sodium salt of a compound of the formula

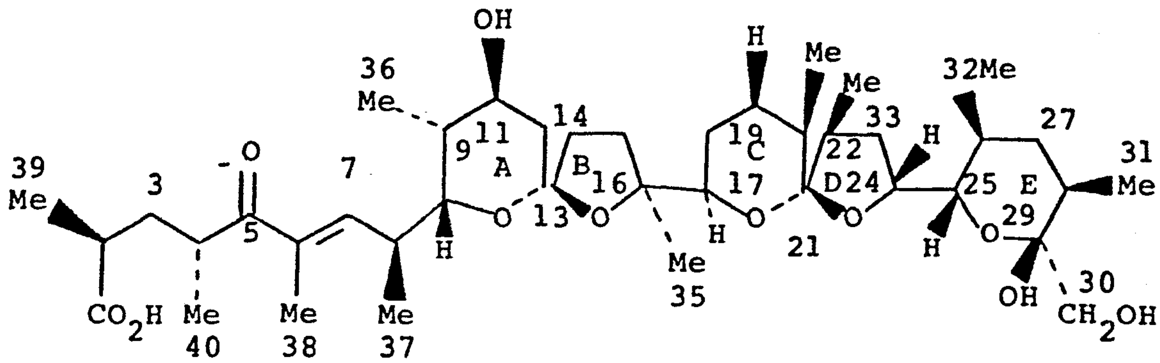

1

comprising the steps of a) concentrating a methylene chloride solution containing <.05% water and the sodium salt of said compound of formula 1 until crystallization commences; b) adding at least an equal volume of dry hexane containing <.05% water to the resulting slurry; and c) filtering and drying the solids.

2. The process of claim 1, wherein the methylene chloride solution is concentrated by heating above the boiling point at atmospheric pressure.

3. A process for preparing a pharmaceutical composition, characterised by mixing a crystalline, anhydrous sodium salt as defined in claim 1 with a pharmaceutically acceptable diluent or carrier.